# FASCICULE DE BREVET EUROPEEN

(11) **EP 3 217 831 B1**
(45) Date de publication et mention de la délivrance du brevet: **26.08.2020**
(21) Numéro de dépôt: 15817544.8
(22) Date de dépôt: 06.11.2015
(51) Int. Cl.: A43B 13/20, A47C 31/12

(54) **SYSTÈME DE RÉGULATION DE PRESSION AGISSANT LOCALEMENT SUR LA PEAU ET LE TISSU SOUS-CUTANÉ**
SYSTEM ZUR EINSTELLUNG VON LOKAL AUF HAUT UND UNTERHAUTGEWEBE EINWIRKENDEN DRUCK
SYSTEM FOR ADJUSTING PRESSURE LOCALLY ACTING ON THE SKIN AND SUBCUTANEOUS TISSUE

(30) Priorité: 10.11.2014 FR 1460869
(43) Date de publication de la demande: 20.09.2017
(73) Titulaire: Ecole Polytechnique Fédérale de Lausanne (EPFL), 1015 Lausanne (CH)
(72) Inventeur: PERRIARD, Yves, CH-2000 Neuchâtel (CH); PATAKY, Zoltan, CH-1196 Gland (CH); GRIVON, Daniel, CH-2000 Neuchâtel (CH); CIVET, Yoan, F-74200 Marin (FR)
(74) Mandataire: Grosfillier, Philippe
(86) Numéro de dépôt international: PCT/IB2015/058574
(87) Numéro de publication internationale: WO 2016/075599

(56) Documents cités:
- CA-C- 2 056 176
- US-A- 5 813 142
- US-A1- 2006 248 750
- US-B2- 7 409 735

## Description

### Domaine de l'invention

L'invention concerne la régulation de pression qui peut s'exercer localement sur la peau et le tissu sous-cutané, par exemple sur la plante du pied. Elle peut être avantageusement utilisée dans le domaine médical ou le domaine sportif.

### Etat de la technique

Les personnes qui souffrent de diabète sucré et de neuropathie peuvent développer des plaies et des ulcères sous la plante du pied. Une des causes de la formation de ces ulcères plantaires est liée à la présence de zones de surpression plantaire qui peuvent être localisées sous toute la plante du pied, le plus souvent au niveau des têtes métatarsiennes.

Actuellement, le traitement des ulcères plantaires chez les patients diabétiques se limite principalement à des systèmes passifs, qui induisent uniquement une décharge de la zone à risque du pied (soit avec un plâtre ou botte plâtrée, chaussure de décharge ou semelle sur mesures, soit avec un système qui permet la création de trous dans une semelle spécifique). Avec ces systèmes, le taux de récidive des ulcères du pied est très élevé car, après la suppression de ces dispositifs thérapeutiques (après guérison), la pression plantaire locale redevient élevée ou le patient développe une ou plusieurs nouvelles zones d'hyperpression plantaire sous une autre région des pieds. Les dispositifs thérapeutiques actuels ne permettent pas la redistribution de la pression plantaire d'une manière équilibrée et régulière, adaptée aux activités de la vie quotidienne des patients.

Il existe quelques systèmes, divulgués notamment dans les demandes de brevet US2003/120353A1 et US2006/0248750A1, qui détectent la pression en divers endroits de la plante du pied ou qui redistribuent automatiquement la pression plantaire.

La demande US2003/120353A1 décrit un module indépendant ayant une raideur variable et contrôlable en réponse à une charge variable. Ce module est constitué de deux chambres disposées verticalement qui contiennent un liquide avec une viscosité variable (magnéto-rhéologique ou électro-rhéologique) et qui sont mises en communication entre elles par un orifice. Le changement de viscosité induit au niveau de l'orifice permet de régler l'écoulement du liquide d'une chambre à l'autre, qui est directement lié à la déformation du module et donc à une réponse différente suivant la charge appliquée.

Dans le système précité on utilise seulement un ou deux modules (de taille relativement importante) qui jouent le rôle d'amortisseurs.

En outre, ce système ne prend pas en considération les problèmes liés au guidage/contrôle mécanique de la déformation verticale des modules. En effet, aucune structure est décrite pour éviter une torsion du module et maintenir constant le diamètre de l*'orifice.* L'absence de soutien du guidage mécanique peut provoquer le blocage du module (à cause des fortes forces de cisaillement qui interviennent au contact du pied au sol) et une déformation non contrôlée de l'orifice.

La demande US2006/0245750A1 décrit une chaussure avec une semelle divisée en chambres souples (coussins) qui sont remplies en particulier avec du liquide rhéologique ayant la capabilité de changer de viscosité si soumis à un champ magnétique/électrique. Les différentes chambres sont reliées entre elles par des *'canaux de communication'* qui permettent le passage du fluide d'un coussin au coussin adjacent. Chaque canal comporte une valve magnéto-rhéologique (électro-rhéologique) capable de changer la viscosité du fluide à l'intérieur du canal, et augmenter ainsi la résistance avec laquelle le liquide peut écouler.

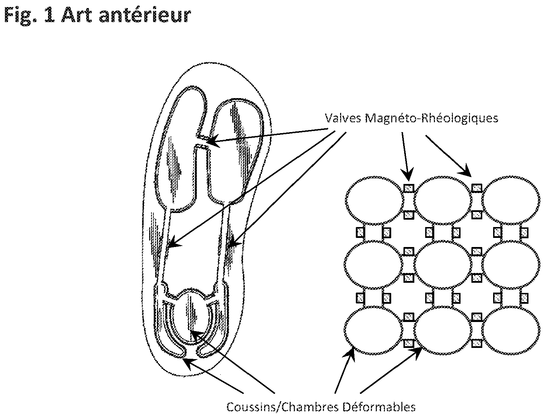

Le fait de disposer les valves à côté des coussins présente de nombreux inconvénients, en particulier :
- Difficulté d'intégration des parties rigides des valves (forcement présente pour pouvoir obtenir une efficacité suffisante du système) avec le reste de la semelle en contact avec le pied et normalement souple.
- Forte réduction de la densité des coussins.
- Augmentation de la complexité du système (p.ex. 9 modules communicants nécessitent 12 valves de contrôle, cf. figure 1).
- Diminution de l'efficacité et la flexibilité du système à cause de la difficulté d'échange de liquide entre les modules qui ne sont pas adjacents.

US 7 409 735 B2 décrit un système de régulation de pression agissant localement sur la peau comprenant un ensemble de modules (10) adjacents distribués de manière à former une couche.

Il existe donc un besoin de réduire, voire d'éliminer, tous ces inconvénients.

### Description de l'invention

Les inconvénients de l'état de la technique sont fortement réduits, voire totalement éliminés, grâce à la présente invention qui concerne un système de régulation de pression agissant localement sur la peau et le tissu sous-cutané. Le système comprend un ensemble de modules adjacents distribués de manière à former une couche ; chaque module comprend les éléments suivants disposés le long d'une même direction : Un coussin déformable comprenant une cavité, une valve, un réservoir et un capteur de pression. La cavité et le réservoir communiquent par le biais de la valve et le capteur est disposé de manière à capter une pression agissant directement ou indirectement sur le coussin. A cet effet, il peut être disposé sur la paroi du coussin (mesure directe) ou à l'intérieur de la cavité. Dans ce 2^{e} cas on mesure la pression du liquide (mesure indirecte). Le système comprenant en outre une boucle de rétroaction disposée de manière à augmenter ou réduire la déformation du coussin en fonction de la pression détectée par le capteur.

Avantageusement, le système selon l'invention comprend plusieurs modules miniaturisés et adjacents qui couvrent la surface de l'avant-pied et/ou du talon. Ils sont disposés avec une densité suffisante pour détecter correctement les différentes zones de surpression. De préférence, le système est capable de modifier la raideur de chacun des modules (donc permettre leur déformation) et ainsi permettre une redistribution.

Le système selon l'invention peut également inclure des moyens de guidage qui contraignent mécaniquement le déplacement d'un organe disposé dans le réservoir (piston ou membrane élastique). En outre, toute force de cisaillement qui peut entrer en jeu dans le contact pied-module est reprise par le coussin sans endommager le fonctionnement du module-même.

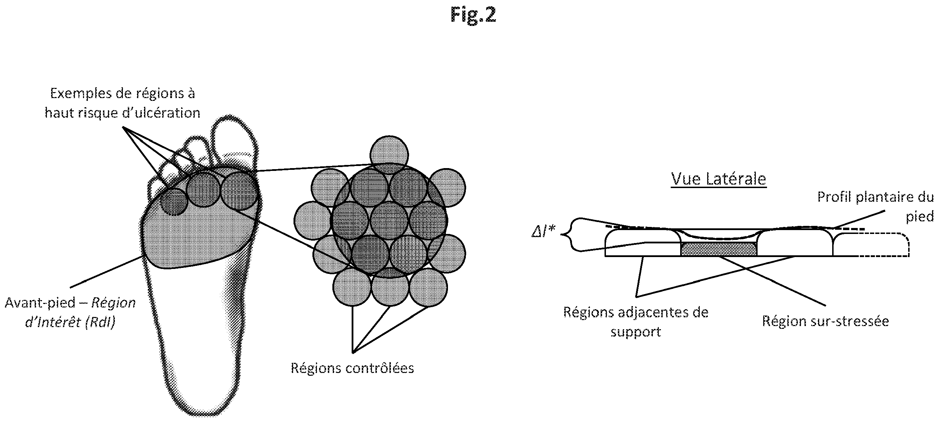

Les modules peuvent être « fluidiquement » indépendants ou communiquer entre eux. Chaque module définit une certaine surface. L'ensemble des modules permet de changer la raideur/souplesse dans différentes zones en dessous de la surface du système, par exemple un pied (Figure 2). Le système selon l'invention résout notamment les problèmes de surpression localisés sous la surface plantaire des pieds. Le système est capable de mesurer et détecter les zones qui présentent des pics de pression et d'adapter sa forme pour redistribuer homogènement cette dernière. Il est donc possible de contrôler la hauteur de chacun des modules pour modifier la forme de la semelle, dynamiquement lorsque le patient se déplace. Le changement des propriétés de chaque module de la semelle (et donc de la semelle elle-même) est effectué par rapport aux informations obtenues par les mesures de pressions de chacun des capteurs présents sur les différents modules.

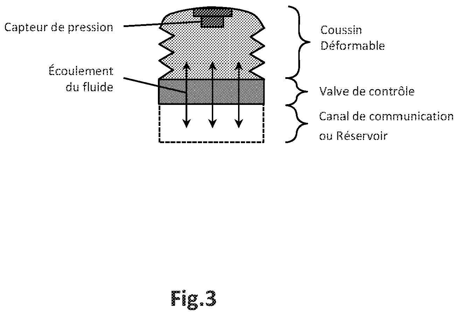

Un module est composé de trois parties principales (Figure 3), à savoir (i) un coussin déformable comportant une cavité destinée à recevoir un fluide, (ii) un réservoir et (iii) une valve de contrôle disposée entre le coussin et le réservoir.

Selon un mode de réalisation de l'invention, le coussin est disposé dans la partie supérieure du module. Il est réalisé avec un matériau souple et déformable. La géométrie du coussin peut être en forme de soufflet. D'autres géométries qui facilitent la déformation dans une direction préférentielle (ici verticale) sont aussi possibles. Le coussin est rempli d'un fluide incompressible (eau, huile, fluide magnéto-rhéologique...). Dans la variante de la figure 3, un capteur de pression est disposé dans la partie supérieure et sur la face interne de la paroi du coussin. Cette mesure permet de déterminer la pression de contact entre le pied du patient et le coussin déformable. D'autres capteurs de pression peuvent aussi être utilisés à l'intérieur ou à l'extérieur du coussin. Toute autre disposition du système de mesure qui permet de déterminer la pression de contact entre le pied et le module-même peut être envisagée comme une possible solution.

La valve contrôle le débit du fluide qui se déplace entre la cavité du coussin et le réservoir. De préférence, la valve est conçue pour occuper la même surface que celle du coussin. Cette disposition verticale des parties principales du module permet de maximiser la densité de modules pour une surface donnée. Plus précisément, toute sorte de valve ayant la capacité de satisfaire les contraintes de dimension, de miniaturisation, de consommation d'énergie, de pression maximale soutenable et de débit maximal admis pour l'application et la configuration (en dessous du coussin déformable) décrites peuvent être considérées comme des solutions possibles.

Le fluide entrant dans le réservoir provient de la cavité du coussin et - si les modules sont fluidiquement connectés - des autres modules.

Le fonctionnement d'un module peut être défini par 3 états:
- Valve fermée : le coussin maintient sa position de départ et reste rigide, le fluide étant incompressible et n'ayant pas la possibilité d' entrer et/ou de sortir de la cavité. Dans ce cas, si une pression externe (p.ex. le pied du patient) s'exerce sur le module, le coussin ne se déforme pas. Le module agit donc comme un élément de semelle rigide.
- Valve ouverte : le coussin peut se déformer car le fluide contenu dans la cavité peut se déplacer dans le réservoir.
- Contrôle de la vitesse d'écoulement du fluide : En agissant au niveau de la valve . Dans ce cas, le module présentera une raideur variable.

Pour un liquide usuel, le contrôle de la vitesse d'écoulement est assuré par une augmentation ou une diminution de l'ouverture de la valve.

Alternativement, on peut utiliser un Fluide Magnéto-Rhéologiques (MRF) et une valve MRF.

Un MRF est constitué d'un liquide non-magnétique (eau ou huile) dans lequel sont dispersées des particules magnétiques (poudre de fer généralement). La principale propriété de ce liquide est sa capacité à changer de viscosité lorsqu'il est soumis à un champ magnétique.

Une valve MRF est composée d'un circuit magnétique et d'un élément créant un champ magnétique (par exemple bobine, aimant...). L'interaction du champ magnétique et du MRF produit un changement de viscosité de ce dernier, et modifie donc son passage à travers la valve. Si l'intensité du champ magnétique est suffisamment haute, le MRF devient visqueux au point que son débit à travers la valve est proche, voire équivalent, de zéro.

Comme indiqué précédemment, les modules peuvent être agencés et connecté de différentes manières.

### I. Modules communicants

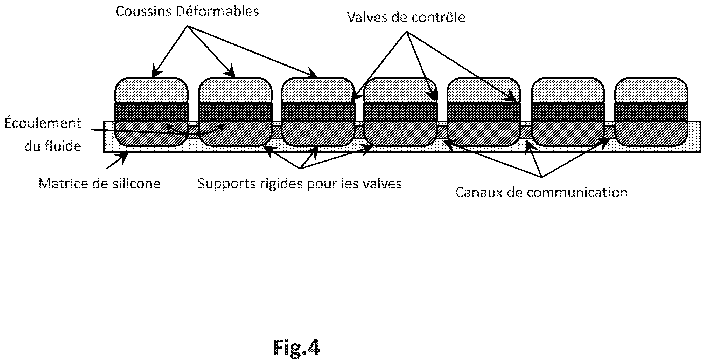

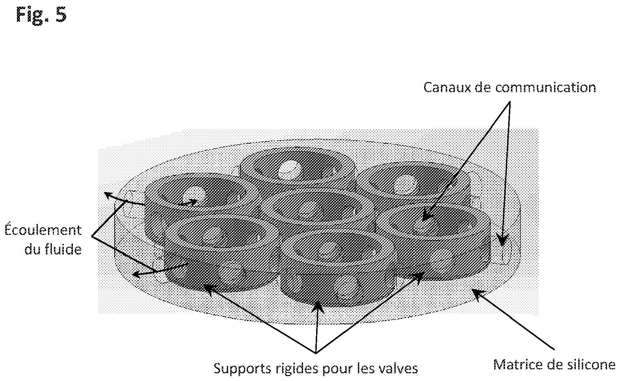

Dans cette configuration, le liquide peut passer d'un module/coussin à un autre à travers un canal de communication. La valve correspondante à chaque module contrôle le passage du fluide du coussin déformable au canal commun. Les Figures 4 et 5 présentent une vue schématique du canal commun et un mode de réalisation.

Cette solution garantit une plus grande flexibilité, un haut niveau d'intégration et une simplicité dans la réalisation du système complet.

### II. Modules indépendants

Dans cette variante, le module constitue un système isolé. La valve régule le passage du liquide entre le coussin et le réservoir, contrôlant donc la hauteur et la raideur du module comme décrit précédemment.

Pour un fonctionnement correct du module, il est nécessaire que, une fois que la pression du pied est retirée du coussin, celui-ci puisse retourner dans sa configuration/hauteur initiale (non-déformée) : il faut donc faire remonter le MRF depuis le réservoir dans le coussin. Pour réaliser cette fonction, deux différentes solutions sont décrites ci-dessous.

### 1. Système avec un piston entraîné par un ressort

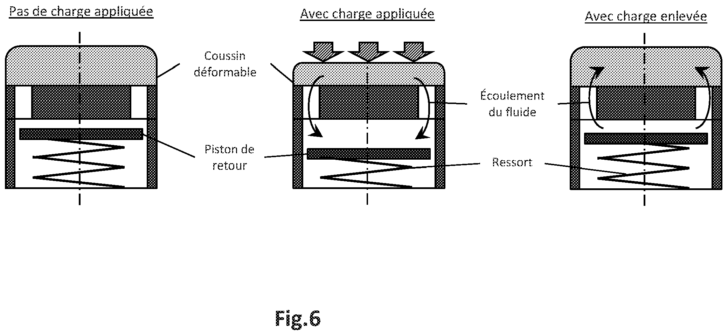

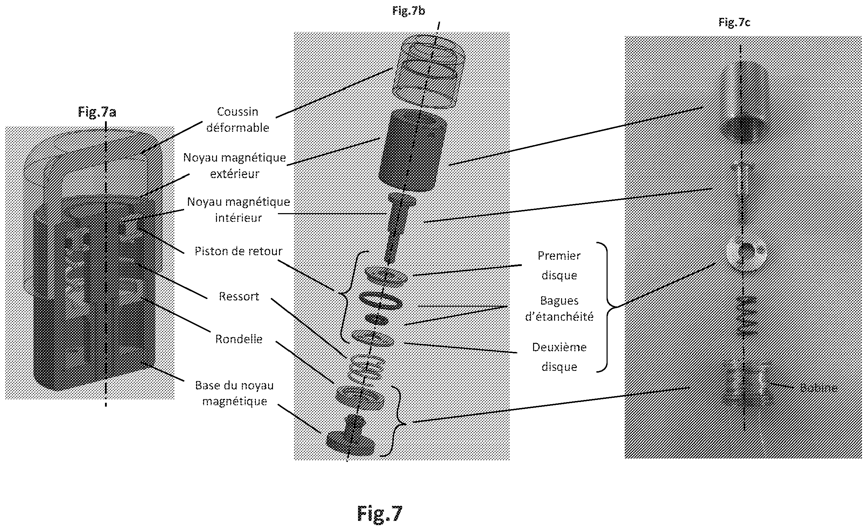

Quand le liquide s'écoule depuis le coussin dans le réservoir à cause de la pression du pied, le piston est poussé en bas et le ressort se comprime. Dès que la pression du pied est enlevée, le ressort fait remonter le liquide dans le coussin (Figures 6 et 7).

Des ressorts de raideurs différentes peuvent être utilisés pour obtenir des effets variables.

### 2. Système avec une membrane élastique déformable en latex

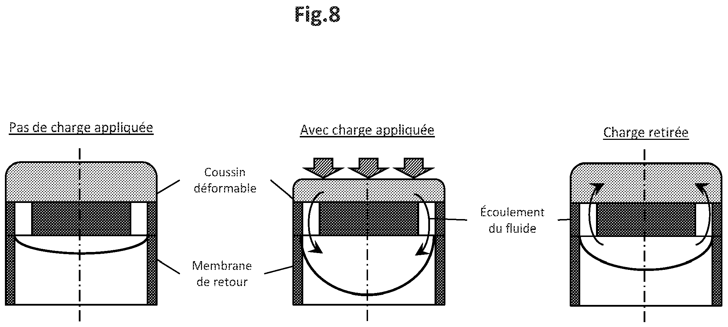

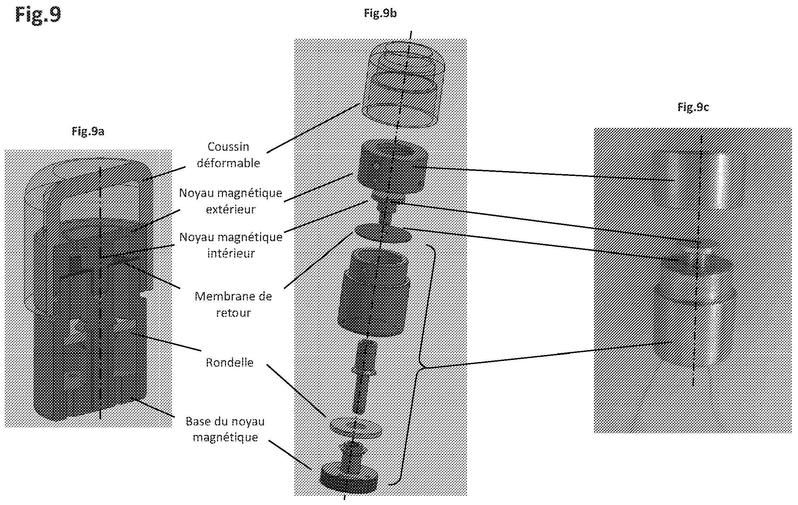

Dans cette variante, une membrane élastique circulaire est positionnée juste en dessous de la valve. Dès que le liquide est poussé en bas à cause de la déformation du coussin , la membrane en latex (ou tout autre élément élastique et étanche) se déforme pour accueillir le liquide. Elle peut se déformer jusqu'à couvrir tout le volume du réservoir. Dès que la pression du pied sur le module est retirée, la membrane retourne dans sa configuration initiale, ce qui fait remonter le liquide vers le coussin (Figures 8 et 9).

Il est possible d'utiliser des membranes d' épaisseurs différentes pour régler la force de rappel qui fait remonter le liquide dans la cavité du coussin.

L'invention ne se limite pas aux exemples présentés dans ce document. N'importe quel module permettant d'assurer une régulation de la pression agissant localement sur la peau et le tissu sous-cutané peut être utilisé.

L'invention n'est également pas limitée à une géométrie, à un agencement ou à des dimensions spécifiques des modules.

## Revendications

1. Système de régulation de pression agissant localement sur la peau comprenant un ensemble de modules adjacents distribués de manière à former une couche; chaque module comprenant un coussin déformable comprenant une cavité, une valve, un réservoir et un capteur de pression ; ces éléments de module étant disposés le long d'une même direction, la cavité et le réservoir communiquant par le biais de la valve et le capteur étant disposé de manière à capter une pression agissant directement ou indirectement sur le coussin ; le système comprenant en outre une boucle de rétroaction disposée de manière à augmenter ou réduire la déformation du coussin en fonction de la pression détectée par le capteur.

2. Système selon la revendication 1 dans lequel chaque module est « fluidiquement » indépendant.

3. Système selon la revendication 1 dans lequel au moins 2 modules sont reliés « fluidiquement ».

4. Système selon l'une quelconque des revendications précédentes dans lequel chaque réservoir comprend un ressort disposé de manière à exercer une force de rappel en direction du coussin.

5. Système selon l'une des revendications 1 à 3 dans lequel chaque réservoir comprend une membrane élastique disposée de manière à exercer une force de rappel en direction du coussin.

6. Système selon l'une quelconque des revendications précédentes dans lequel chaque réservoir/cavité comprend une fluide de type MRF.

7. Système selon l'une quelconque des revendications précédentes dans lequel le capteur est disposé sur la face externe de la paroi du coussin, de manière à mesurer directement une pression externe qui agit sur le coussin.

8. Système selon l'une quelconque des revendications 1 à 6 dans lequel le capteur est disposé dans la cavité du coussin, de manière à mesurer la pression qui règne à l'intérieur de celle-ci.

9. Système selon l'une quelconque des revendications précédentes, dans lequel l'ensemble des modules est dimensionné de manière à couvrir la surface d'un avant-pied et/ou d'un talon.

## Patentansprüche

1. System zur Einstellung von Druck, der lokal auf die Haut einwirkt, beinhaltend eine Anordnung von benachbarten Modulen, die so verteilt sind, dass sie eine Schicht bilden; wobei jedes Modul ein verformbares Kissen, das einen Hohlraum beinhaltet, ein Ventil, einen Speicher und einen Drucksensor beinhaltet; wobei diese Modulelemente entlang einer gleichen Richtung angeordnet sind, wobei der Hohlraum und der Speicher über das Ventil miteinander kommunizieren und der Sensor so angeordnet ist, dass er einen direkt oder indirekt auf das Kissen einwirkenden Druck erfasst; wobei das System ferner eine Feedbackschleife beinhaltet, die so angeordnet ist, dass sie die Verformung des Kissens in Abhängigkeit von dem durch den Sensor erfassten Druck erhöht oder verringert.

2. System nach Anspruch 1, wobei jedes Modul "fluidisch" unabhängig ist.

3. System nach Anspruch 1, wobei mindestens 2 Module "fluidisch" verbunden sind.

4. System nach einem der vorhergehenden Ansprüche, wobei jeder Speicher eine Feder beinhaltet, die so angeordnet ist, dass sie eine Rückstellkraft in Richtung des Kissens ausübt.

5. System nach einem der Ansprüche 1 bis 3, wobei jeder Speicher eine elastische Membran beinhaltet, die so angeordnet ist, dass sie eine Rückstellkraft in Richtung des Kissens ausübt.

6. System nach einem der vorhergehenden Ansprüche, wobei jeder Speicher/Hohlraum ein Fluid vom Typ MRF beinhaltet.

7. System nach einem der vorhergehenden Ansprüche, wobei der Sensor auf der Außenseite der Wand des Kissens angeordnet ist, um einen Außendruck, der auf das Kissen einwirkt, direkt zu messen.

8. System nach einem der Ansprüche 1 bis 6, wobei der Sensor in dem Hohlraum des Kissens angeordnet ist, um den Druck, der im Inneren desselben herrscht, zu messen.

9. System nach einem der vorhergehenden Ansprüche, wobei die Anordnung der Module so dimensioniert ist, dass sie die Oberfläche eines Vorfußes und/oder einer Ferse bedeckt.

## Claims

1. System for regulating pressure acting locally on the skin, said system comprising a set of adjacent modules distributed in such a way as to form a layer; each module comprising a deformable cushion having a cavity, a valve, a reservoir, and a pressure sensor; these module elements being arranged along a same direction, the cavity and the reservoir communicating by way of the valve, and the sensor being arranged in such a way as to detect a pressure acting directly or indirectly on the cushion; the system additionally comprising a feedback loop arranged in such a way as to increase or reduce the deformation of the cushion according to the pressure detected by the sensor.

2. System according to Claim 1, in which each module is "fluidically" independent.

3. System according to Claim 1, in which at least two modules are connected "fluidically".

4. System according to any one of the preceding claims, in which each reservoir comprises a spring which is arranged in such a way as to exert a restoring force in the direction of the cushion.

5. System according to one of Claims 1 through 3, in which each reservoir comprises an elastic membrane which is arranged in such a way as to exert a restoring force in the direction of the cushion.

6. System according to any one of the preceding claims, in which each reservoir/cavity comprises a fluid of the MRF type.

7. System according to any one of the preceding claims, in which the sensor is arranged on the outer face of the wall of the cushion, in such a way as to measure directly an external pressure that acts on the cushion.

8. System according to any one of Claims 1 through 6, in which the sensor is arranged in the cavity of the cushion, in such a way as to measure the pressure that prevails inside said cavity.

9. System according to any one of the preceding claims, in which the set of modules is dimensioned in such a way as to cover the surface of a forefoot and/or of a heel.
